# EUROPEAN PATENT APPLICATION

(11) **EP 4 371 604 A1**
(43) Date of publication of application: **22.05.2024**
(21) Application number: 22207744.8
(22) Date of filing: 16.11.2022
(51) Int. Cl.: A61N 1/08, A61N 1/37, G01R 29/08, G01R 31/00, A61N 1/372

(54) **ELECTRONIC DEVICE ENABLING SAFE OPERATION OF AN IMPLANTABLE MEDICAL DEVICE**

(71) Applicant: BIOTRONIK SE & Co. KG, 12359 Berlin (DE)
(72) Inventor: Dörr, Thomas, 12437 Berlin (DE); Lang, Volker, 12161 Berlin (DE); Diem, Björn Henrik, 14197 Berlin (DE)
(74) Representative: Biotronik Corporate Services SE

(57) **Abstract**

The present invention relates to an electronic device (4) emitting an alternating electric or magnetic field during its operation, the electronic device (4) comprising a detection and control unit (5) capable of detecting a presence of an implantable medical device (2) within a first distance (7) to the electronic device. According to an aspect of the invention, the detection and control unit (5) is configured to automatically cause a performance of at least one of a plurality task upon detecting an implantable medical device (2) within the first distance (7). Examples of these tasks are: reducing an electric and/or magnetic power of the electronic device (4); deactivating a component assembly or functional entity of the electronic device (4) in dependence on a factual or expected electric or magnetic field emitted by the component assembly or functional entity; and reducing a maximum power of a component assembly or functional entity of the electronic device (4) in dependence on a factual or expected electric or magnetic field emitted by the component assembly or functional entity upon operating the component assembly or functional entity with a nonreduced maximum power.

## Description

The present invention relates to an electronic device according to the preamble of claim 1, to a detection and control unit for an electronic device according to the preamble of claim 9, to a method for controlling an operation of an electronic device according to the preamble of claim 10, and to an arrangement comprising an electronic device according to the preamble of claim 14.

Whenever an implantable medical device comes into proximity with an electronic device, an interference between both devices may occur. Such interference can impart the functions of the implantable medical device and can thus be relevant for the medical safety of a patient carrying the implantable medical device and approaching an electronic device.

US 8,761,717 B1 describes the possibility to detect an implanted medical device within an effective proximity of an electronic device by a so-called safety control module coupled with the electronic device. According to this US patent, the performance of a function of the electronic device can be disabled and only enabled again after having received a resolution from an operator of the electronic device.

EP 2 380 627 A1 describes a control device having a first interface configured to wirelessly communicate with an implant and a second interface configured to communicate with a machine such as a vehicle. The first interface receives physiologic data or a suitability index from the implant describing the fitness of a patient wearing the implant to operate the machine. The second interface transmits the physiological data or suitability index together with a unique identifier to the machine control system. Consequently, an operation of the machine will only be possible if the patient wearing the implantable medical device has a sufficient physiologic suitability to do so.

It is an object of the present invention to provide an electronic device, the operation of which can be adjusted more flexible than according to prior art solutions upon the detection of an implantable medical device in effective proximity to the electronic device to enable a safe operation of the implantable medical device also in proximity with the electronic device.

This object is achieved with an electronic device having the features of claim 1. Such an electronic device emits an alternating electric or magnetic field during its operation. The electronic device comprises a detection and control unit that is capable of detecting a presence of an implantable medical device within a first distance to the electronic device. Typically, the implantable medical device is detected in an implanted state, i.e., in a state in which the implantable medical device is implanted to a patient.

According to an aspect of the present invention, the detection and control unit is configured to automatically cause a performance of at least one of the following tasks after having detected an implantable medical device within the first distance: reducing an electric and/or magnetic power of the electronic device; deactivating a component assembly or functional entity of the electronic device in dependence on a factual or expected electric or magnetic field emitted by the component assembly or functional entity; reducing a maximum power of a component assembly or functional entity of the electronic device in dependence on a factual or expected electric or magnetic field emitted by the component assembly or functional entity upon operating the component assembly or functional entity with a non-reduced maximum power; controlling a stepping motor of the electronic device with a reduced pulsing (this will reduce critical interference frequencies); deactivating a radio communication unit of the electronic device or operatively coupled with the electronic device; reducing a transmission power of a radio communication unit of the electronic device or operatively coupled with the electronic device; changing an adjustment of an antenna of a radio communication unit of the electronic device or operatively coupled with the electronic device; charging an accumulator of the electronic device with a reduced electric power; operating the electronic device with a maximum power that guarantees that a specific absorption rate (SAR) of energy due to the emitted electric or magnetic field is below an admissible threshold (e.g., if the electronic device is a medical device such as a magnetic resonance tomography (MRT) device, the maximum power of which is dependent on the chosen parameter combination); reducing a threshold value at which a protective appliance or a deactivating appliance of the electronic device is activated; preventing an application of a predefinable (critical) frequency modulation method; disabling an operational mode that may provoke an interference with a function of the detected implantable device; activating a protective assembly of the electronic device and/or a protective device operatively connected to the electronic device, wherein the activation of the protective assembly and/or of the protective device increases a minimum required distance between a user and at least a part of the electronic device; and generating an alert signal to be output (e.g., to the patient carrying the implantable medical device).

Due to the adaptation of the functionality of the electronic device upon detection of an implantable medical device within the first distance (i.e., within effective proximity to the electronic device), possible limitations for patients carrying an implantable medical device are reduced, in particular in a professional context or during the operation of high-performing electric vehicles.

In an embodiment, the first distance is a distance lying in a range of from 10 cm to 10 m, in particular from 20 cm to 9 m, in particular from 30 cm to 8 m, in particular from 40 cm to 7 m, in particular from 50 cm to 6 m, in particular from 60 cm to 5 m, in particular from 70 cm to 4 m, in particular from 80 cm to 3 m, in particular from 90 cm to 2 m, in particular from 1 m to 1.5 m.

In an embodiment, the electronic device comprises a communication unit. This communication unit enables a direct or indirect wireless communication with the implantable medical device. The communication is, in an embodiment, an active communication with an implantable medical device that is detected within the first distance to the electronic device. A direct communication between the implantable medical device and the electronic device is a communication without any additional communication unit, e.g., without relay station or broadcasting station. An indirect communication between the implantable medical device and the electronic device is a communication via at least one additional communication device such as a relay station or a broadcasting station.

In an embodiment, the relay station is realized in form of a patient device or a smart phone of the patient. Then, a specific communication app can be executed on the smartphone of the patient and can use the resources of the smart phone to establish an indirect communication between the implantable medical device and the electronic device via the smartphone of the patient.

All standard data transmission protocols or specifications are appropriate for such a wireless data communication. Examples of standard data transmission protocols or specifications are the Medical Device Radiocommunications Service (MICS), the Bluetooth Low Energy (BLE) protocol, the Zigbee specification, the long range wide area network (LoRaWAN) protocol, the wireless personal area network (WPAN) specification, the low-power wide-area network (LPWAN) specification, the wireless local area network (WLAN) specification, the Global System for Mobile Communications (GSM) specification, the Long-Term Evolution (LTE) standard, and the fifth-generation technology standard for broadband cellular networks (5G).

In an embodiment, the detection and control unit is configured to detect at least two different types of implantable medical devices. Furthermore, in this embodiment, the detection and control unit is configured to distinguish between the at least two different types of implantable medical devices and to adapt an operational parameter of the task to be performed depending on the type of the detected implantable medical device. The type of the detected implantable medical device may be transferred to the detection and control unit via a unique identifier of the implantable medical device. Such a unique identifier can be transmitted from the implantable medical device to the communication unit of the electronic device.

In an embodiment, the electronic device comprises a memory unit that is operatively coupled with the detection and control unit. In this context, the memory unit comprises stored data on unique identifiers of different types of implantable medical devices. If a unique identifier is transferred from an implantable medical device, e.g., via the communication unit of the electronic device, to the electronic device, it can then be compared with the data stored in the memory unit of the electronic device. Based on this comparison, the type of implantable medical device and its susceptibility to malfunctions due to interference with the electronic device can be determined and the functions of the electronic device that are considered to be relevant for an interference with the specific implantable medical device can be adjusted to avoid such interference.

In an embodiment, the electronic device is chosen from the group consisting of industrial plants, machine tools, industrial robots, vehicles, safety gates, medical devices, radio systems, electrical detection systems, generators, eroding machines, electrolysis systems, electroplating systems, magnetic separators, magnetizing/demagnetizing equipment, annealing/hardening/tempering equipment, induction heating equipment, ball bearing heating equipment, melting furnaces (electric arc or inductive), brazing equipment, crack testing equipment, pipe end testing equipment, high-frequency plastic punching machines, high-frequency dryers, resistance-welding equipment, capacitor-welding equipment, and high-frequency plastic welding equipment.

In an embodiment, the electronic device comprises a feedback unit. This feedback unit serves for signaling to a user whether the electronic device has detected the presence of an implantable medical device. Then, it is immediately apparent for the user whether there is an implantable medical device within the first distance to the electronic device. If the user wears an implantable medical device by himself or herself, the feedback unit may help to assure the user that the implantable medical device has been properly detected and that certain functions of the electronic device have been adjusted due to this detection. This enables a particularly user friendly and safe operation of the electronic device.

In an embodiment, the detection and control unit is configured to quantitatively determine a distance between the electronic device and the implantable medical device. In this embodiment, the detection and control unit is further configured to adapt an operational parameter of the task to be performed depending on the determined distance. This functionality of the electronic device allows a very specific and finely tunable adjustment of the operational parameters of the electronic device. While certain operational parameters need to be only slightly adjusted if an implantable medical device is detected within the first distance, but still quite far away from the electronic device, a more pronounced adjustment of the operational parameters may be necessary in case that the implantable medical device comes closer to the electronic device. Thus, this embodiment does not only allow a qualitative assessment whether or not an implantable medical device has been entered an area covering the first distance from the electronic device, but can rather determine how far the detected implantable medical device is located from the electronic device. Furthermore, this embodiment enables a distance-dependent adjustment of functional parameters of the electronic device so that both the safe operation of the implantable medical device is made possible and only minimum restrictions for the operation of the electronic device are necessary.

In an embodiment, the alternating electric or magnetic field emitted during the (intended, i.e. normal) operation of the electronic device exceeds - at least as regards to peak values - a threshold defined by the standard E DIN VDE 0848-3-1. Typically, only electronic devices emitting strong electromagnetic fields lying above the threshold defined by the beforementioned standard are harmful to the operation of implantable medical devices. Therefore, only the functionality of such electronic devices needs to be adapted to guarantee safe operation of implantable medical devices coming into effective proximity to the electronic device.

In an aspect, the present invention relates to a detection and control unit for an electronic device that emits an alternating electric or magnetic field during its operation. In this context, the detection and control unit is capable of detecting the presence of an implantable medical device within a first distance to the detection and control unit. Furthermore, the detection and control unit comprises an interface configured to transmit a control signal to an electronic device.

According to this aspect of the invention, the control signal causes, in an electronic device operatively coupled to the detection and control unit, a performance of at least one of the following tasks upon detecting an implantable medical device within the first distance: reducing an electric and/or magnetic power of the electronic device; deactivating a component assembly or functional entity of the electronic device in dependence on a factual or expected electric or magnetic field emitted by the component assembly or functional entity; reducing a maximum power of a component assembly or functional entity of the electronic device in dependence on a factual or expected electric or magnetic field emitted by the component assembly or functional entity upon operating the component assembly or functional entity with a non-reduced maximum power; controlling a stepping motor of the electronic device with a reduced pulsing (this will reduce critical interference frequencies); deactivating a radio communication unit of the electronic device or operatively coupled with the electronic device; reducing a transmission power of a radio communication unit of the electronic device or operatively coupled with the electronic device; changing an adjustment of an antenna of a radio communication unit of the electronic device or operatively coupled with the electronic device; charging an accumulator of the electronic device with a reduced electric power; operating the electronic device with a maximum power that guarantees that a specific absorption rate (SAR) of energy due to the emitted electric or magnetic field is below an admissible threshold (e.g., if the electronic device is a medical device such as a magnetic resonance tomography (MRT) device, the maximum power of which is dependent on the chosen parameter combination); reducing a threshold value at which a protective appliance or a deactivating appliance of the electronic device is activated; preventing an application of a predefinable (critical) frequency modulation method; disabling an operational mode that may provoke an interference with a function of the detected implantable device; activating a protective assembly of the electronic device and/or a protective device operatively connected to the electronic device, wherein the activation of the protective assembly and/or of the protective device increases a minimum required distance between a user and at least a part of the electronic device; and generating an alert signal to be output (e.g., to the patient carrying the implantable medical device).

In an aspect, the present invention relates to a method for controlling the operation of an electronic device emitting an alternating electric or magnetic field during its operation, in particular for controlling an operation of an electronic device according to the preceding explanations. This method comprises the steps explained in the following.

In one method step, a presence of an implantable medical device within a first distance to the electronic device is detected with the detection and control unit of the electronic device.

In another method step, a performance of at least one of the following tasks is automatically caused upon detecting an implantable medical device within the first distance: reducing an electric and/or magnetic power of the electronic device; deactivating a component assembly or functional entity of the electronic device in dependence on a factual or expected electric or magnetic field emitted by the component assembly or functional entity; reducing a maximum power of a component assembly or functional entity of the electronic device in dependence on a factual or expected electric or magnetic field emitted by the component assembly or functional entity upon operating the component assembly or functional entity with a non-reduced maximum power; controlling a stepping motor of the electronic device with a reduced pulsing (this will reduce critical interference frequencies); deactivating a radio communication unit of the electronic device or operatively coupled with the electronic device; reducing a transmission power of a radio communication unit of the electronic device or operatively coupled with the electronic device; changing an adjustment of an antenna of a radio communication unit of the electronic device or operatively coupled with the electronic device; charging an accumulator of the electronic device with a reduced electric power; operating the electronic device with a maximum power that guarantees that a specific absorption rate (SAR) of energy due to the emitted electric or magnetic field is below an admissible threshold (e.g., if the electronic device is a medical device such as a magnetic resonance tomography (MRT) device, the maximum power of which is dependent on the chosen parameter combination); reducing a threshold value at which a protective appliance or a deactivating appliance of the electronic device is activated; preventing an application of a predefinable (critical) frequency modulation method; disabling an operational mode that may provoke an interference with a function of the detected implantable device; activating a protective assembly of the electronic device and/or a protective device operatively connected to the electronic device, wherein the activation of the protective assembly and/or of the protective device increases a minimum required distance between a user and at least a part of the electronic device; and generating an alert signal to be output (e.g., to the patient carrying the implantable medical device).

In an embodiment, a communication between a communication unit of the electronic device and the detected implantable medical device is established. As outlined above, such communication enables the transmission of additional data between the detected implantable medical device and the electronic device. In an embodiment, such data includes information on the specific type of the implantable medical device that has been detected.

In an embodiment, the communication between the communication unit and the detected implantable medical device is established via a third communication device. This third communication device is, in an embodiment, a cloud device or a network device that has access to a database. This database comprises, in an embodiment, data on specific electronic devices and specific implantable medical devices that are allowed to communicate with each other. In this embodiment, the third communication device can actively prevent or allow a communication between the electronic device and the implantable medical device. This enhances the security level of the communication pathway between the electronic device and the implantable medical device.

In an embodiment, the third communication device allows the communication between the communication unit and the detected implantable medical device only if the third communication device has successfully authenticated at least one of the communication unit and the detected implantable medical device. Without such successful authentication, the communication between the communication unit of the electronic device and the detected implantable medical device will be prevented. This guarantees that the electronic device does not negatively influence the functionality of the implantable medical device (e.g., as part of a hacking attack directed against the implantable medical device). At the same time, the third communication device effectively prevents a non-authenticated influence of the electronic device by an implantable medical device that is possibly not intended to adjust the functionalities of the electronic device.

In an aspect, the present invention relates to an arrangement comprising an electronic device according to the preceding explanations and an implantable medical device. In this context, the detection and control unit of the electronic device is capable of detecting a presence of the implantable medical device within the first distance to the electronic device.

In an embodiment, the implantable medical device is chosen from the group consisting of implantable pulse generators (IPGs), implantable cardioverter-defibrillators (ICDs), devices for cardiac resynchronization (CRT), such as CRT defibrillators (CRT-D) or CRT pacemakers (CRT-P), any kind of neurostimulators, electrocardiogram (ECG) loop or event recorders, ventricular assist devices, implantable sensors and/or non-implantable sensors (as for instance pressure sensors or biochemical sensors), implantable hearing aids, active orthopedic implants, and drug pumps.

All embodiments of the electronic device can be combined with each other in any way and can be transferred either individually or in any arbitrary combination to the detection and control unit, to the method of operating the electronic device, and to the arrangement. Likewise, all embodiments of the detection and control unit can be combined in any way and can be transferred either individually or in any arbitrary combination to the electronic device, to the method of operating the electronic device, and to the arrangement. Furthermore, all embodiments of the method for operating the electronic device can be combined in any way and can be transferred either individually or in any arbitrary combination to the electronic device, to the detection and control unit, and to the arrangement. Finally, all embodiments of the arrangement can be combined in any way and can be transferred either individually or in any arbitrary combination to the electronic device, to the detection and control unit, and to the method for operating the electronic device.

Further details of aspects of the present invention will be explained in the following referring to an exemplary embodiment and accompanying Figures. In the Figures:

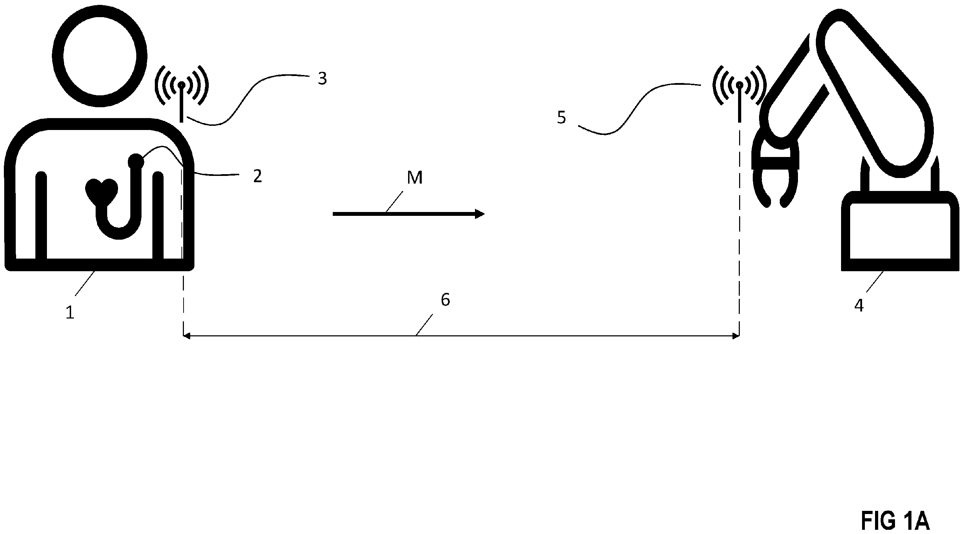

- Figure 1A: shows an arrangement of an electronic device and an implantable medical device in a first condition and

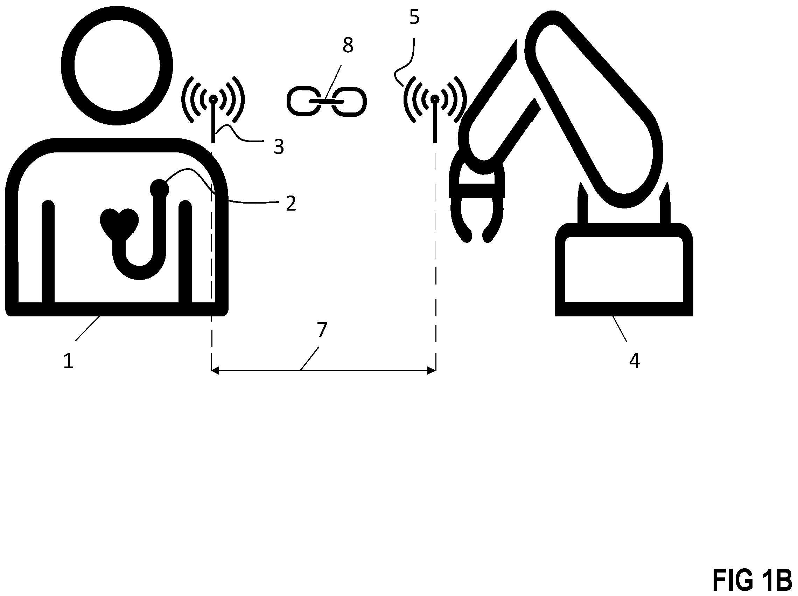

- Figure 1B: shows the arrangement of Figure 1A in a second condition.

Figure 1A shows a patient 1 carrying an implantable pulse generator (IPG) 2 as example for an implantable medical device in an implanted state. The IPG 2 comprises a communication unit 3 that can also be denoted as wireless interface.

Figure 1A furthermore shows an electronic device 4 that emits an electric or magnetic field during its normal operation. The electronic device 4 can perform different tasks, wherein the operational parameters, under which these tasks are performed, define a strength of the alternating electrical magnetic field emitted during operation of the electronic device 4. The electronic device 4 comprises a detection and control unit 5 that is generally able to detect the presence of an implantable medical device such as the IPG 2 if the implantable medical device is located within effective proximity to the electronic device 4. In the situation shown in Figure 1A, the patient 1 and therewith the communication unit 3 of the IPG 2 is located at a second distance 6 to the detection and control unit 5 of the electronic device 4. This second distance 6 is, however, bigger than a distance that is considered to constitute an effective proximity to the electronic device 4. Therefore, the detection and control device 5 does not detect the presence of the IPG 2 and thus of the patient 1.

If the patient 1 now moves closer to the electronic device in the direction of the arrow marked with an M, the patient 1 comes into effective proximity with the electronic device 4. This situation is depicted in Figure IB. In this situation, the communication unit 3 of the IPG 2 is within a first distance 7 to the detection and control unit 5 of the electronic device 4. Thus, the first distance 7 is a comparatively small distance at which the patient 1 or the IPG 2 is in effective proximity to the electronic device 4. The detection and control unit 5 therefore detects the presence of the IPG 2. Furthermore, it is possible that a data link 8 is established between the communication unit 3 and a communication unit forming part of the electronic device 4 or of the detection and control unit 5 of the electronic device 4. By such a data link 8, it is possible to exchange data on the type of the IPG 2. The electronic device can then - by referring back to data stored in a memory unit of the electronic device 4 - decide which operational parameters of the electronic device are to be adjusted so that an operation of the electronic device 4 does not impart the functionality of the IPG 2 even though the patient 1 is located within the first distance 7 to the electronic device 4. This significantly enhances the patient safety and operational reliability of the IPG 2, even though the patient 1 is in effective proximity to the electronic device 4 and thus within an area in which alternating electric or magnetic fields are typically emitted by the electronic device 4. However, due to an adjustment of (specific) operational parameters of the electronic device 4, these alternating electric or magnetic fields are attenuated such that no severe interference between the electronic device 4 and the IPG 2 will occur any longer. As a result, the patient 1 can operate the electronic device 4 basically without any limitation regarding the health status of the patient 1 and the operability of the IPG 2.

## Claims

1. Electronic device (4) emitting an alternating electric or magnetic field during its operation, the electronic device (4) comprising a detection and control unit (5) capable of detecting a presence of an implantable medical device (2) within a first distance (7) to the electronic device,
**characterized**
**in that** the detection and control unit (5) is configured to automatically cause a performance of at least one of the following tasks upon detecting an implantable medical device (2) within the first distance (7): reducing an electric and/or magnetic power of the electronic device (4); deactivating a component assembly or functional entity of the electronic device (4) in dependence on a factual or expected electric or magnetic field emitted by the component assembly or functional entity; reducing a maximum power of a component assembly or functional entity of the electronic device (4) in dependence on a factual or expected electric or magnetic field emitted by the component assembly or functional entity upon operating the component assembly or functional entity with a non-reduced maximum power; controlling a stepping motor of the electronic device (4) with a reduced pulsing; deactivating a radio communication unit of the electronic device (4) or operatively coupled with the electronic device (4); reducing a transmission power of a radio communication unit of the electronic device (4) or operatively coupled with the electronic device (4); changing an adjustment of an antenna of a radio communication unit of the electronic device (4) or operatively coupled with the electronic device (4); charging an accumulator of the electronic device (4) with a reduced electric power; operating the electronic device (4) with a maximum power that guarantees that a specific absorption rate of energy due to the emitted electric or magnetic field is below an admissible threshold; reducing a threshold value at which a protective appliance or a deactivating appliance of the electronic device (4) is activated; preventing an application of a predefinable frequency modulation method; disabling an operational mode that may provoke an interference with a function of the detected implantable device (2); activating a protective assembly of the electronic device (4) and/or a protective device operatively connected to the electronic device (4), wherein the activation of the protective assembly and/or of the protective device increases a minimum required distance between a user and at least a part of the electronic device (4); and generating an alert signal to be output.

2. Electronic device according to claim 1, **characterized in that** the electronic device (4) comprises a communication unit enabling a direct or indirect communication (8) with an implantable medical device (2).

3. Electronic device according to claim 1 or 2, **characterized in that** the detection and control unit (5) is configured to detect at least two different types of implantable medical devices (2), to distinguish between the at least two different types of implantable medical devices (2), and to adapt an operational parameter of the task to be performed in dependence on the type of the detected implantable medical device (2).

4. Electronic device according to any of the preceding claims, **characterized in that** the electronic device (4) comprises a memory unit operatively coupled with the detection and control unit (5), wherein the memory unit comprises stored data on unique identifiers of different types of implantable medical devices (4).

5. Electronic device according to any of the preceding claims, **characterized in that** the electronic device is chosen from the group consisting of industrial plants, machine tools, industrial robots, vehicles, safety gates, medical devices, radio systems, electrical detection systems, generators, eroding machines, electrolysis systems, electroplating systems, magnetic separators, magnetizing/demagnetizing equipment, annealing/hardening/tempering equipment, induction heating equipment, ball bearing heating equipment, melting furnaces, brazing equipment, crack testing equipment, pipe end testing equipment, high-frequency plastic punching machines, high-frequency dryers, resistance-welding equipment, capacitor-welding equipment, and high-frequency plastic welding equipment.

6. Electronic device according to any of the preceding claims, **characterized in that** the electronic device (4) comprises a feedback unit configured to signalize to a user whether the electronic device (4) has detected the presence of an implantable medical device (2).

7. Electronic device according to any of the preceding claims, **characterized in that** the detection and control unit (5) is configured to quantitatively determine a distance between the electronic device (4) and the implantable medical device (2) and to adapt an operational parameter of the task to be performed in dependence on the determined distance.

8. Electronic device according to any of the preceding claims, **characterized in that** the alternating electric or magnetic field emitted during the operation of the electronic device (4) exceeds a threshold defined by the standard E DIN VDE 0848-3-1.

9. Detection and control unit for an electronic device that emits an alternating electric or magnetic field during its operation, wherein the detection and control unit (5) is capable of detecting a presence of an implantable medical device (2) within a first distance (7) to the detection and control unit (5), wherein the detection and control unit (5) comprises an interface configured to transmit a control signal to an electronic device (4),
**characterized**
**in that** the control signal causes, in an electronic device (4) operatively coupled to the detection and control unit (5), a performance of at least one of the following tasks upon detecting an implantable medical device (2) within the first distance (7): reducing an electric and/or magnetic power of the electronic device (4); deactivating a component assembly or functional entity of the electronic device (4) in dependence on a factual or expected electric or magnetic field emitted by the component assembly or functional entity; reducing a maximum power of a component assembly or functional entity of the electronic device (4) in dependence on a factual or expected electric or magnetic field emitted by the component assembly or functional entity upon operating the component assembly or functional entity with a non-reduced maximum power; controlling a stepping motor of the electronic device (4) with a reduced pulsing; deactivating a radio communication unit of the electronic device (4) or operatively coupled with the electronic device (4); reducing a transmission power of a radio communication unit of the electronic device (4) or operatively coupled with the electronic device (4); changing an adjustment of an antenna of a radio communication unit of the electronic device (4) or operatively coupled with the electronic device (4); charging an accumulator of the electronic device (4) with a reduced electric power; operating the electronic device (4) with a maximum power that guarantees that a specific absorption rate of energy due to the emitted electric or magnetic field is below an admissible threshold; reducing a threshold value at which a protective appliance or a deactivating appliance of the electronic device (4) is activated; preventing an application of a predefinable frequency modulation method; disabling an operational mode that may provoke an interference with a function of the detected implantable device (2); activating a protective assembly of the electronic device (4) and/or a protective device operatively connected to the electronic device (4), wherein the activation of the protective assembly and/or of the protective device increases a minimum required distance between a user and at least a part of the electronic device (4); and generating an alert signal to be output.

10. Method for controlling an operation of an electronic device (4) emitting an alternating electric or magnetic field during its operation, in particular an electronic device (4) according to any of the preceding claims, the method comprising the following steps:
a) detecting, with a detection and control unit (5) of the electronic device (4), a presence of an implantable medical device (2) within a first distance (7) to the electronic device (4),
b) automatically causing a performance of at least one of the following tasks upon detecting an implantable medical device (2) within the first distance (7): reducing an electric and/or magnetic power of the electronic device (4); deactivating a component assembly or functional entity of the electronic device (4) in dependence on a factual or expected electric or magnetic field emitted by the component assembly or functional entity; reducing a maximum power of a component assembly or functional entity of the electronic device (4) in dependence on a factual or expected electric or magnetic field emitted by the component assembly or functional entity upon operating the component assembly or functional entity with a non-reduced maximum power; controlling a stepping motor of the electronic device (4) with a reduced pulsing; deactivating a radio communication unit of the electronic device (4) or operatively coupled with the electronic device (4); reducing a transmission power of a radio communication unit of the electronic device (4) or operatively coupled with the electronic device (4); changing an adjustment of an antenna of a radio communication unit of the electronic device (4) or operatively coupled with the electronic device (4); charging an accumulator of the electronic device (4) with a reduced electric power; operating the electronic device (4) with a maximum power that guarantees that a specific absorption rate of energy due to the emitted electric or magnetic field is below an admissible threshold; reducing a threshold value at which a protective appliance or a deactivating appliance of the electronic device (4) is activated; preventing an application of a predefinable frequency modulation method; disabling an operational mode that may provoke an interference with a function of the detected implantable device (2); activating a protective assembly of the electronic device (4) and/or a protective device operatively connected to the electronic device (4), wherein the activation of the protective assembly and/or of the protective device increases a minimum required distance between a user and at least a part of the electronic device (4); and generating an alert signal to be output.

11. Method according to claim 10, **characterized in that** a communication (8) between a communication unit of the electronic device (4) and the detected implantable medical device (2) is established.

12. Method according to claim 11, **characterized in that** the communication (8) between the communication unit and the detected implantable medical device (2) is established via a third communication device.

13. Method according to claim 12, **characterized in that** the third communication device allows the communication (8) between the communication unit and the detected implantable medical device (2) only if the third communication device has successfully authenticated at least one of the communication unit and the detected implantable medical device (2).

14. Arrangement comprising an electronic device according to any of claims 1 to 8 and an implantable medical device (2), wherein the detection and control unit (5) of the electronic device (4) is capable of detecting a presence of the implantable medical device (2) within a first distance (7) to the electronic device (4).

15. Arrangement according to claim 14, **characterized in that** the implantable medical device (4) is chosen from the group consisting of implantable pulse generators, implantable cardioverter-defibrillators, devices for cardiac resynchronization, neurostimulators, electrocardiogram loop or event recorders, ventricular assist devices, implantable sensors, non-implantable sensors, implantable hearing aids, active orthopedic implants, and drug pumps.
